# EUROPEAN PATENT APPLICATION

(11) **EP 3 467 149 A1**
(43) Date of publication of application: **10.04.2019**
(21) Application number: 17809474.4
(22) Date of filing: 02.03.2017
(51) Int. Cl.: C23C 22/07, B05D 5/02, A61L 27/32

(54) **METHOD FOR THE NANOMETRIC DEPOSITION OF CALCIUM PHOSPHATE ON THE SURFACE OF AN ANODIZED TITANIUM IMPLANT**

(30) Priority: 06.06.2016 BR 10201612926
(71) Applicant: Brunella Sily De Assis, Bumachar, Vila Velha, CEP 29102121 (BR); Serafim, Alexandre José, CEP.: 29055-090 Vitória ES (BR)
(72) Inventor: MUÑIZ FERREIRA, José Ricardo, Valinhos CEP 13278-141 (BR); NAVARRO DA ROCHA, Daniel, Rio de Janeiro CEP 22231-150 (BR); PRADO DA SILVA, Marcelo Henrique, Ipanema CEP 22420-041 (BR); BLAZUTTI MARCAL, Rubens Lincoln, Ipanema CEP 22210-000 (BR)
(74) Representative: Felber, Josef
(86) International application number: PCT/BR2017/000020
(87) International publication number: WO 2017/210757

(57) **Abstract**

NANOMETRIC CALCIUM PHOSPHATE DEPOSITION PROCESS ON ANODIZED TITANIUM IMPLANT SURFACE is a uniform and homogeneous nanometric calcium phosphate deposition method over the entire surface of anodized titanium implants and provides a chemical bond at implant-tissue interface; this process can be performed throughout the implant, or partially, in specific areas of its surface, without modification in macrogeometry and microroughness of the implant, and provides calcium and phosphate ions on the anodized titanium implant surfaces, and provides a unique nanometric morphology, increasing the specific surface area and modifying the hydrophobic behavior of the anodized titanium implant surface, converting it in highly hydrophilic and bioactive.

## Description

The present invention relates to deposition method of nanometric calcium phosphate on anodized titanium implant surface, producing a rich calcium and phosphate ions, highly bioactive and hydrophilic surface used in biomedical field.

Nowadays, the success in intraosseous implant surgery is determined by osseointegration phenomena. Firstly, the main objective of intraosseous implant system is to achieve the primary stabilization, guaranteed by an intimate physical contact between implant and bone tissue. However, the implant stability is initially decreased up to three weeks due to bone remodeling and followed by an increase to the baseline at 4-5 weeks. Concurrently, the secondary stability is defined by bone modeling and remodeling started on the implant surface, allowing functional loading of implant and long-term implant success. Bioactive surfaces have been developed to shorten the healing bone periods on the osseoconductive implant surface by secondary bone deposition, reducing the implant stability dip. Furthermore, it has been a consensus of researches that such surface optimization may accelerate the contact osteogenesis and thereby allowing immediate or early loading protocols.

Bioactive materials, such as glass-ceramics and calcium phosphate, have high chemical affinity with bone tissue, which provide the capability of direct bonding to living bone, so-called bioactivity. Bioactive surfaces show osseoconductivity, stimulating the production of osteoblasts and thereby accelerate the process of transformation of woven bone into lamellar bone (LEGEROS, 2008). Although commercial intraosseous implants present excellent biocompatibility and biomechanics, these materials are bioinert and cannot stimulate a chemical bone-implant interface and, therefore, interact weakly with the biological environment. It is therefore expected that new demands of intraosseous implants are designed by controlling of chemical reactivity of their surfaces in the body environment. A method to further enhancing the biological response and chemically bonding bone-implant interface is the use of calcium phosphate coating (BOSCO *et al,* 2012). In addition, calcium phosphates favor the proteins adsorption on their surface, which can mediate a superior binding of osteoprogenitor cells when compared to untreated implant surfaces (KILPADI *et al,* 2001).

From the end of 1990s to date, the literature has been demonstrated that osseointegration is improved and accelerated through several methods of modifications on the implant surface such as sand blasting, acid etching and anodic oxidation. Today, titanium implant surfaces with moderately textured microtopographies (Sa between 1 and 2 µm) is provided for the major of commercially available implants due to the beneficial interaction between the implant and biological tissues (US 5456723 A, WO2004008983A1, US 8251700 B2). The scientific literature has extensively described that after intraosseous implantation, a direct contact between implant and bone tissue will biomechanically stabilize the implant system (BRANEMARK, 1978). Morphological surface modifications of intraosseous implant, as porosity and roughness, have been demonstrated that a higher bone deposition and better mechanical stability in the initial moments of bone healing, when compared to untreated titanium implant surfaces. (GITTENS *et al,* 2011, JAVED & ROMANOS 2010, KAMMERER *et al,* 2012, OLISCOVICZ *et al,* 2013). An adequate microroughness surface have an increase on surface area that allow the stabilization of fibrin clot during osteogenic cells migration to the implant surface for subsequent bone deposition directly on its surface.

Calcium phosphates have been demonstrated great potential for use in bone regeneration and coating titanium implant surfaces. Several surface modifications and calcium phosphates coating methods are verified in the literature. However, there have been indications that the application of a thick calcium phosphate coatings cause clinical complications, such as coating fractures and eventual inflammation caused by delamination of ceramic particles. These complications have contributed to discrediting the first commercial coating method - plasma-spray. Currently, the coating thickness by the plasma spray technique can range from 10 to 200 µm (US8632843B2, US5603338, US5863201, US6652765, US20100187172, EP0407698A1, CN102051569A). Therefore, in order to improve the mechanical properties of coated dental implant, nanometric calcium phosphate is an alternative method to mimic the biological environment and the nanostructural architecture of mineral bone. Nanoroughness implant surface have shown improvement in cellular responses and higher adhesion of osteoblasts cells and mineral deposition of calcium and phosphate ions on the surface (US20070110890).

There are several techniques for the synthesis of hydroxyapatite powders with nanometric particle size (US8287914B2, SADAT-SHOJAI et al, 2013). However, there are few techniques with a controlled nanometer thickness of calcium phosphate coating over the entire titanium implant surface at low cost. Dip coating is an example of nanometric deposition method which uses surfactants in order to obtain dispersed hydroxyapatites particles into a solution before substrate coating. The production of a suitable solution (microemulsion) is not trivial and can cause a poor adhesion or non-uniforms coatings.

Regarding of above information from public knowledge, as well as proposing a quite new approach to optimizing the anodized titanium implant surfaces with calcium and phosphate ions, higher specific surface area and wettability, a nanometric calcium phosphate deposition on anodized titanium implant was developed. The present invention avoids the biomechanical complications of micrometer thickness of calcium phosphate coatings with the control of calcium phosphate layer deposition ranging 10 and 200 nm by a simple adaptation of calcium and phosphate concentration. The absence of surfactants or a previous step of hydroxyapatite powder synthesis show the simple and low cost production of the invention when compared to other techniques.

The nanometric calcium phosphate deposition process on anodized titanium implant surface, as well as its results, may be better explained and illustrated through the detailed description in accordance with the following attached figures:
FIGURE 01 shows the electromicrographies of the anodized titanium surface on an nanometric scale.
FIGURE 02 shows the electromicrographies of the nanometric calcium phosphate deposition on the anodized titanium surface on an nanometric scale.
FIGURE 03 shows the electromicrographies of the nanometric calcium phosphate deposition on the anodized titanium surface exhibiting the preserved surface microstructure.
FIGURE 04 shows the EDS mapping, confirming the elements: calcium and phosphorous after nanometric calcium phosphate deposition process on anodized titanium implant surface.
FIGURE 05 shows the hydrophobic behavior of anodized titanium implant surface after 30 days of air exposure.
FIGURE 06 shows the hydrophilic behavior of nanometric calcium phosphate deposition process on anodized titanium implant surface after 30 days of air exposure.

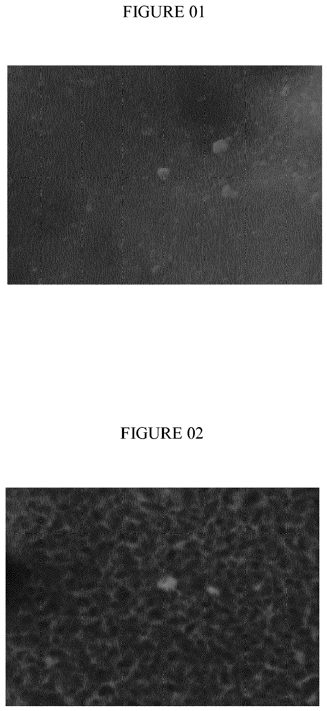

According to the figures above, the nanometric calcium phosphate deposition process on anodized titanium implant surface provided a unique nanometric morphology (Figure 2) that increase the specific surface area and modify the hydrophobic behavior of anodized titanium implant surface (control) to hydrophilic and bioactive surface. The deposition of three-dimensional nanometric calcium phosphate exhibiting nano needle-like morphology, ranging from 10 to 200 nm of thickness and lengths, are randomly disposed on the entire surface. The immersion step of anodized titanium implant surface into rich calcium and phosphate ions solution can be performed on the entire or partially surface, resulting in an uninterrupted and controlled nanometric calcium phosphate deposition without modification in the microstructure design of anodized titanium implant. Therefore, the anodized titanium implant surface may be designed to exhibit a hydrophobic behavior in the coronal third of the implant body while the middle and apical third of implant show hydrophilic behavior to optimizing the osseointegration. Thus, the nanometric calcium phosphate deposition process provides a high bioactivity on the anodized titanium implant surface and, thereby, hastening the rate of osseointegration at early stages.

According to Sessile-drop method, wettability property of biomaterial surfaces can be determined by the measurement of contact angle. The present invention exhibits a contact angle less than 5° at time t = 0 seconds (height of the first contact of the droplet with the surface). This behavior is typical for highly hydrophilic surfaces. An inert atmosphere (such as: oxygen, argon, nitrogen, noble gases or a mixture of these gases) for the storage of intraosseous implant is recommended due to a substantial maintenance of hydrophilic behavior when the exposure to air is avoided. The deposition of nanometric calcium phosphate on anodized titanium implant surfaces demonstrated hydrophilic property even after 30 days exposed to air.

There are three variables in the calcium phosphate deposition process: immersion in rich calcium and phosphate ions solution, temperature and immersion time in alkaline solution, described below.

The deposition method is performed by immersion of the anodized titanium implant into calcium and phosphate ions solutions in order to obtain a nanometric calcium phosphate layer.

The stabilization of calcium and phosphate ions solution can be performed by reagents, such: lactic acid, acetic acid, citric acid, uric acid, as well as the use of polyalcohol, surfactants and chelating agents, such as: EDTA, DPPE, PEG, glycerol, sorbitol, xylitol, among others. These reagents will be volatilized after heat treatment between 50 and 400 °C.

For example, the rich in calcium and phosphate ions solution is prepared by mixing 0.5M calcium hydroxide to 1M lactic acid and, thereafter, 0.3M ortho-phosphoric acid is slowly added to the above mixture. The control of calcium and phosphate ions concentration into solution will define the thickness of coating; for example, the lower the concentration, the thinner the calcium phosphate coating. Calcium and phosphate ions concentration and Ca/P molar ratio into solution are variable; preferentially, Ca/P molar ratio is between 1 and 2; for example: approximately, 1.67.

Before immersion step, the implant may undergo a thermochemical treatment in order to increase the specific surface area and wettability property of the anodized titanium implant surface. For example, an acid solution treatment (HCl, H₂SO₄, HNO₃, HF, H₃PO₄, CaCl₂ and/or mixture of these reagents) with subsequent alkaline solution treatment (NaOH, KOH, NH₄OH and/or mixture of these reagents) at several temperatures, for example: room temperature to 100 °C) and times (ranging from 1 to 180 min). Molarity of acid and alkaline solution may vary from 0.01 to 1M. Temperature is a factor that directly depends on the concentration used in the chemical bath, and can vary between 30 °C and 150 °C. Preferentially, the higher temperature, the lower the calcium and phosphate concentration. Time is a factor that depends on both concentration and temperature; preferentially, the higher temperature and concentration, the lower the time; for example, time ranging from 1 to 180 min. First, an alkaline solution followed by an acidic solution treatment can be performed to obtain a suitable wettability property for the immersion step of the anodized titanium implant.

The immersion step of intraosseous implant into rich calcium and phosphate ions solution is controlled by parameters, such: speed immersion and emersion, immersion time and post-process rest. The anodized titanium implant can be totally or partially immersed in the solution for total or partial surface deposition. At this step, during or after immersion of the implant, a vacuum of 10 to 10⁻⁵ mbar may be performed to remove the air bubbles from microstructure of anodized titanium implant surface and, thus, allow the contact of solution with the whole implant surface. In this step, if the vacuum is performed during the immersion, a higher concentration of calcium and phosphorus ions will be available within the implant microroughness when compared to microsmooth surface area. Thus, the calcium phosphate precipitation will be observed within the microroughness. A heating bath can also be performed during immersion of intraosseous implant in a rich calcium and phosphate ions solution, since homogeneous or heterogeneous nucleation does not occur in the implant-solution system.

After the immersion step, anodized titanium implants will be dried at room temperature, thus a uniform nanometric calcium phosphate coating over the entire surface of the anodized titanium implant is obtained without modification of its microstructure. The drying step of intraosseous implant can be performed in vacuum for up to 15 min followed by a second oven drying for 10 min at 60°C. After the drying step, a heat treatment may also be carried out between 50 and 300 ° C for a time between 1 and 150 min. This heat treatment step leads to a morphology modification of nanometric calcium phosphate coating when compared to the nanometric calcium phosphate coating obtained without this treatment step. Preferentially, vacuum is performed after immersion step of the intraosseous implant in rich calcium and phosphate ions solution and the heating treatment step is performed between 50 and 300 °C for a time between 1 and 150 min.

After the drying and heat treatment step, the intraosseous implant is immersed in alkaline solution (KOH, NaOH, NH₄OH, among other strong bases, and/or mixture of these reagents) under thermochemical bath. The molarity of alkaline solution may vary from 0.01 to 1 Molar. Temperature is a factor that directly depends on the concentration used in the chemical bath, and can vary between 30 °C and 150 °C. Preferentially, the higher temperature, the lower the alkaline solution concentration. Time is a factor that depends on both concentration and temperature; preferentially, the higher temperature and concentration, the lower the time; for example, time ranging from 1 to 180 min. Preferentially, intraosseous implant is immersed into an alkaline solution of 0.01M, 0.05M or 0.1M KOH for the time of 30 min to 1 hour under thermochemical treatment between 30 °C and 100 °C. The final step of calcium phosphate deposition process consists of a heat treatment for the consolidation of calcium phosphate phase and crystallinity control. The heat treatment can vary between 300 °C and 700 °C for the time 1 second to 60 minutes.

According to Figures 01 and 02, the homogeneous and uniform modification in the entire nanoscale surface of anodized titanium implant is verified, without modification in the microsurface.

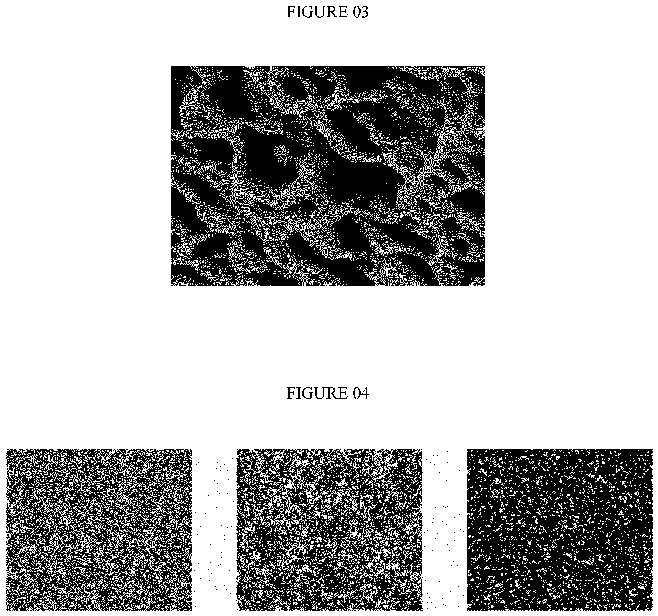

While Figure 3 shows the preserved micromorphology of the anodized surface after the nanometric calcium phosphate deposition process, Figure 4 confirms the uniformly presence of calcium and phosphorus ions throughout the implant surface.

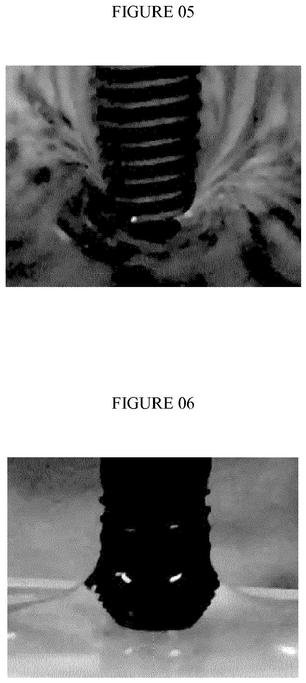

While Figure 5 shows the hydrophobicity of the anodized titanium implant surface, Figure 06 demonstrates the hydrophilicity after the nanometric calcium phosphate deposition on the anodized titanium implant surface by the method described in this invention.

The main advantage of the present invention is the control of nanometric deposition of calcium phosphate in the entire anodized titanium implant surface. The invention demonstrates a novel method to obtain a uniform and homogeneous nanometric features throughout the implant surface by a simple and low cost production process.

The implant surface, after deposition method, shows high wettability and nanometric features throughout the implant surface, even in submicrometric pores. The nanometric and chemical control obtained by this calcium phosphate deposition process on anodized titanium implant surface provide an increased specific surface area, optimization of cellular interaction and chemical bonding between implant surface and bone tissue.

## Claims

1. NANOMETRIC CALCIUM PHOSPHATE DEPOSITION PROCESS ON ANODIZED TITANIUM IMPLANT SURFACE, **characterized by** the fact that the surface possess a nanometric calcium phosphate coating on its surface, and comprises the following steps; deposition process of nanometric calcium phosphate on titanium anodized titanium implant, over the entire surface or a selected area; Anodized titanium implant immersion into a calcium and phosphate rich ions solution that must have Ca/P molar ratio between 1 and 1,67; after immersion, the anodized titanium implants dries at room temperature, under vacuum for 15 minutes, thus obtaining a thin and uniform coating of nanometric calcium phosphate covering the anodized titanium implant surface homogeneously, with no changes on its macro and microroughness; after drying, a heat treatment step is performed where temperature in 150 °C for a time of 15 min; anodized titanium implant immersion into a potassium hydroxide solution for a period between 1 and 180 minutes with concentration varying from 0.01M to 1M and treatment temperature could vary from 30 °C to 150 °C; after this step, the anodized titanium implant surface must exhibit a nanometric calcium phosphate coating with thickness under 200 nanometers; in sequence, anodized titanium implant washing with deionized water until elimination of free potassium on anodized titanium implant surface;

2. NANOMETRIC CALCIUM PHOSPHATE DEPOSITION PROCESS ON ANODIZED TITANIUM IMPLANT SURFACE, according to claim 1, **characterized by** the fact that the anodized titanium implant is made of titanium;

3. NANOMETRIC CALCIUM PHOSPHATE DEPOSITION PROCESS ON ANODIZED TITANIUM IMPLANT SURFACE, according to claim 1, **characterized by** the fact that the titanium intraosseous implant possesses an anodized surface treatment for microroughness creation;

4. NANOMETRIC CALCIUM PHOSPHATE DEPOSITION PROCESS ON ANODIZED TITANIUM IMPLANT SURFACE, according to claim 1, **characterized by** the fact that the anodized titanium implant is used on medical, dentistry, orthopedic and esthetic areas;

5. NANOMETRIC CALCIUM PHOSPHATE DEPOSITION PROCESS ON ANODIZED TITANIUM IMPLANT SURFACE, according to claim 1, **characterized by** the fact that the anodized titanium implant coated with calcium phosphate, Ca/P molar ratio could vary from 1 to 1,67;

6. NANOMETRIC CALCIUM PHOSPHATE DEPOSITION PROCESS ON ANODIZED TITANIUM IMPLANT SURFACE, according to claim 1, **characterized by** the fact that the thermochemical alkali treatment molar concentration could vary from 0.01M to 1M;

7. NANOMETRIC CALCIUM PHOSPHATE DEPOSITION PROCESS ON ANODIZED TITANIUM IMPLANT SURFACE, according to claim 1, **characterized by** the fact that the anodized titanium implant calcium phosphate coating thickness is less then 200 nanometers;

8. NANOMETRIC CALCIUM PHOSPHATE DEPOSITION PROCESS ON ANODIZED TITANIUM IMPLANT SURFACE, according to claim 1, **characterized by** the fact that the anodized titanium implant coated with calcium phosphate shows a high hydrophilicity;

9. NANOMETRIC CALCIUM PHOSPHATE DEPOSITION PROCESS ON ANODIZED TITANIUM IMPLANT SURFACE, according to claim 1, **characterized by** the fact that the anodized titanium implant coated with calcium phosphate contain, on its surface, Ca²⁺ions uniformly available;

10. NANOMETRIC CALCIUM PHOSPHATE DEPOSITION PROCESS ON ANODIZED TITANIUM IMPLANT SURFACE, **characterized by** the fact that the surface possess a nanometric calcium phosphate coating on its surface, and comprises the following steps; deposition process of nanometric calcium phosphate on titanium anodized titanium implant, over the entire surface or a selected area; Anodized titanium implant immersion into a calcium and phosphate rich ions solution that must have Ca/P molar ratio between 1 and 1,67; after immersion, the anodized titanium implants dries at room temperature, under vacuum for 15 minutes, thus obtaining a thin and uniform coating of nanometric calcium phosphate covering the anodized titanium implant surface homogeneously, with no changes on its macro and microroughness; after drying, a heat treatment step is performed where temperature in 150 °C for a time of 15 min; anodized titanium implant immersion into a potassium hydroxide solution for a period between 1 and 180 minutes with concentration varying from 0.01M to 1M and treatment temperature could vary from 30 °C to 150 °C; in sequence, washing with deionized water until elimination of free potassium on anodized titanium implant surface; at the end, the anodized titanium implant coated with calcium phosphate suffer a heat treatment between 300 °C and 700 °C for a time that could vary from 1 second to 60 minutes; after this step, the anodized titanium implant surface must exhibit a nanometric calcium phosphate coating with thickness under 200 nanometers

11. NANOMETRIC CALCIUM PHOSPHATE DEPOSITION PROCESS ON ANODIZED TITANIUM IMPLANT SURFACE, according to claim 10, **characterized by** the fact that the anodized titanium implant is made of titanium;

12. NANOMETRIC CALCIUM PHOSPHATE DEPOSITION PROCESS ON ANODIZED TITANIUM IMPLANT SURFACE, according to claim 10, **characterized by** the fact that the titanium intraosseous implant possesses an anodized surface treatment for microroughness creation;

13. NANOMETRIC CALCIUM PHOSPHATE DEPOSITION PROCESS ON ANODIZED TITANIUM IMPLANT SURFACE, according to claim 10, **characterized by** the fact that the anodized titanium implant is used on medical, dentistry, orthopedic and esthetic areas;

14. NANOMETRIC CALCIUM PHOSPHATE DEPOSITION PROCESS ON ANODIZED TITANIUM IMPLANT SURFACE, according to claim 10, **characterized by** the fact that the anodized titanium implant coated with calcium phosphate, Ca/P molar ratio could vary from 1 to 1,67;

15. NANOMETRIC CALCIUM PHOSPHATE DEPOSITION PROCESS ON ANODIZED TITANIUM IMPLANT SURFACE, according to claim 10, **characterized by** the fact that the thermochemical alkali treatment molar concentration could vary from 0.01M to 1M;

16. NANOMETRIC CALCIUM PHOSPHATE DEPOSITION PROCESS ON ANODIZED TITANIUM IMPLANT SURFACE, according to claim 10, **characterized by** the fact that the anodized titanium implant calcium phosphate coating thickness is less then 200 nanometers;

17. NANOMETRIC CALCIUM PHOSPHATE DEPOSITION PROCESS ON ANODIZED TITANIUM IMPLANT SURFACE, according to claim 10, **characterized by** the fact that the anodized titanium implant coated with calcium phosphate shows a high hydrophilicity;

18. NANOMETRIC CALCIUM PHOSPHATE DEPOSITION PROCESS ON ANODIZED TITANIUM IMPLANT SURFACE, according to claim 10, **characterized by** the fact that the anodized titanium implant coated with calcium phosphate contain, on its surface, Ca²⁺ ions uniformly available;
